# DEMANDE DE BREVET EUROPEEN

(11) **EP 2 098 258 A1**
(43) Date de publication de la demande: **09.09.2009**
(21) Numéro de dépôt: 08405063.2
(22) Date de dépôt: 03.03.2008
(51) Int. Cl.: A61M 5/145, A61M 5/24, B67B 7/92

(54) **Dispositif de distribution d'un liquide d'injection et procédé de fabrication d'une cartouche**

(71) Demandeur: Rochat, Jean-Denis, 1272 Genolier (CH)
(72) Inventeur: Rochat, Jean-Denis, 1272 Genolier (CH)
(74) Mandataire: Savoye, Jean-Paul

(57) **Abrégé**

Ce dispositif de distribution d'un liquide d'injection, comprend un corps tubulaire (9a) présentant à une extrémité une ouverture de sortie (2d). Une cartouche (2) hermétique et jetable contient le liquide d'injection (1), le corps tubulaire (9a) comprend un piston (4) pour expulser le liquide d'injection (1) de la cartouche (2) à travers l'ouverture de sortie (2d), au moins une partie (2b) de la paroi de la cartouche (2) étant déformable sous la pression exercée par le piston (4). Cette cartouche (2) comprend un élément de raccordement étanche (2d) pour un conduit de distribution (3) et un élément de rupture (2e) pour mettre en communication l'intérieur de la cartouche (2) avec le conduit de distribution (3), le corps tubulaire (9a) comportant une partie amovible (9b) pour la mise en place et l'enlèvement de la cartouche (2).

## Description

La présente invention se rapporte à un dispositif de distribution d'un liquide d'injection, comprenant un corps tubulaire présentant à une extrémité une ouverture de sortie et dans lequel une cartouche hermétique et jetable contient le liquide d'injection, ainsi qu'à un procédé de fabrication de la cartouche contenant le liquide d'injection.

Les dispositifs constitués d'une seringue jetable et d'un dispositif d'entraînement connu sous le nom de « pousse-seringues » sont connues et utilisées à grande échelle dans le domaine médical pour l'injection de médicaments, de produits anesthésiant et analgésiques.

Le concept classique comporte une seringue généralement en polypropylène ou polyéthylène, elle-même constituée d'un piston et d'un cylindre. Le piston peut comporter un joint d'étanchéité glissant en caoutchouc ou en silicone lubrifié. Cette seringue est généralement remplie manuellement par transfert depuis une fiole ou une ampoule en verre à l'aide d'une aiguille de gros diamètre, qu'il faut sortir d'un tiroir, extraire d'un emballage stérile, connecter à la seringue de manière stérile, puis transvaser, éliminer l'air, jeter l'aiguille, connecter la seringue de manière stérile à la ligne de perfusion et purger celle-ci.

D'une part, toutes ces manipulations sont faites sur le site opératoire ou hospitalier par un médecin ou une infirmière et engendrent une perte de temps importante et donc des coûts conséquents. D'autre part, le nombre d'éléments stériles consommés est important puisqu'il faut faire le transfert de la fiole jusqu'à une seringue au travers d'une aiguille gros diamètre.

Enfin, du point de vue de la sécurité, l'importance de la manipulation augmente le risque d'erreur. Il est impératif que chaque injection déterminée soit effectuée pour le patient prévu, à la dose prévue, en temps voulu, et notifiée dans le journal prévu à cet effet.

On a déjà proposé de remédier à ces manipulations en utilisant une cartouche pré-remplie de liquide d'injection dans le US 2007/0167912 A1. Cette cartouche est en fait une seringue jetable pré-remplie, comprenant un corps tubulaire, un piston monté dans le corps tubulaire par une extrémité ouverte du corps tubulaire, un joint d'étanchéité entre le piston et le corps tubulaire, un opercule fermant l'autre extrémité du corps tubulaire et un élément pour retenir l'opercule de manière étanche sur le corps tubulaire.

Pour injecter le liquide de la seringue, il faut une aiguille pour perforer l'opercule et des moyens de connexion de cette aiguille avec un conduit d'injection. Même si cette solution permet de supprimer les nombreuses manipulations susmentionnées, la cartouche est composée de plusieurs pièces assemblées. Donc le coût de fabrication de la cartouche jetable est loin d'être négligeable.

Le but de la présente invention est de réduire au maximum la manipulation nécessaire pour préparer l'injection. Elle vise également à réduire le nombre de pièces du dispositif, en particulier le nombre de pièces jetables permettant de réduire le coût de fabrication du dispositif et en particulier celui de sa partie jetable.

A cet effet, la présente invention a tout d'abord pour objet un dispositif de distribution d'un liquide d'injection selon la revendication 1. Elle a également pour objet un procédé de fabrication de la cartouche hermétique et jetable qui contient le liquide d'injection pour ce dispositif selon la revendication 9.

La cartouche du dispositif de distribution objet de l'invention est en une seule pièce sans organe mobile ni joint et peut donc être produite à un coût extrêmement faible, notamment par la technique de conditionnement stérile connue sous le terme anglais de blow-fill-seal qui est une technique combinant l'extrusion soufflage au remplissage suivi de la fermeture hermétique. La mise en place de la cartouche et sa mise en service sont des opérations faciles, rapides et non délicates à effectuer.

Avantageusement, la paroi déformable de la cartouche est sa paroi latérale, susceptible d'être repliée sur elle-même par le déplacement du piston, le segment de la paroi latérale de ce piston, adjacent à la cartouche, présentant un rayon réduit de la valeur d'au moins deux fois l'épaisseur de la paroi latérale.

De préférence, le dispositif est à actionnement par moteur électrique et comporte une batterie d'alimentation.

Avantageusement, l'élément de raccordement comporte une saillie creuse située au centre d'une des parois d'extrémité de la cartouche et qui se termine par un obturateur, une saignée circulaire d'affaiblissement étant formée entre la saillie creuse et l'obturateur.

D'autres particularités et avantages apparaîtront à la lecture de la description qui suit et des dessins qui l'accompagnent et qui illustrent, schématiquement et à titre d'exemple, une forme d'exécution du dispositif de distribution d'un liquide d'injection objet de l'invention.
La figure 1 est une vue en coupe axiale de cette forme d'exécution illustrée dans une première position;
la figure 2 est une vue semblable à la figure 1 illustrée dans une seconde position;
la figure 3 est une vue en perspective éclatée de cette forme d'exécution;
la figure 4 est une vue partielle à plus grande échelle de la figure 2;
la figure 5 est une vue en perspective partielle avec coupe d'un détail des figures 1 et 2.

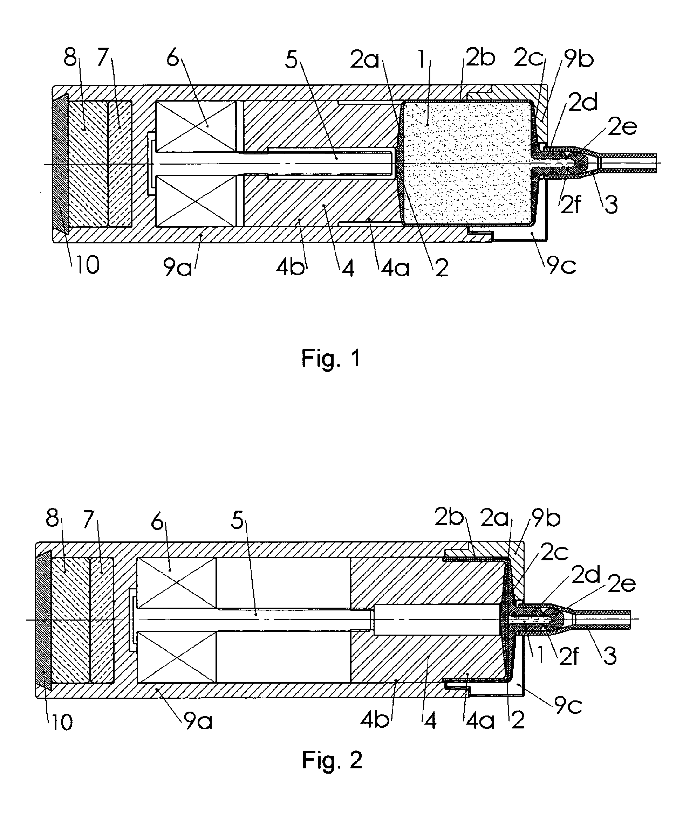

Le dispositif de distribution d'un liquide d'injection illustré par la figure 1 comporte un corps tubulaire 9a fermé à une extrémité par un couvercle 9b muni d'une ouverture radiale 9c, fixé avantageusement au corps tubulaire 9a par une fermeture de type baïonnette. A son autre extrémité, le corps tubulaire est fermé par un couvercle 10, fixé à cran et retenant une batterie électrique d'alimentation 8 et un module électronique 7 comportant un circuit de commande.

Ce corps tubulaire 9a renferme encore un moteur électrique 6 alimenté par la batterie 8. L'arbre d'entraînement 5 du moteur 6 est constitué par un arbre fileté 5 en prise avec un filetage axial d'un piston 4 dont une partie arrière 4b de la paroi latérale est montée coulissante dans le corps tubulaire 9a.

L'extrémité avant du piston 4, opposée au moteur 6 est en contact avec une face d'extrémité 2a d'une cartouche 2 de liquide d'injection 1, dont la face opposée 2c est en contact avec le couvercle 9b fermant l'extrémité avant du corps tubulaire 9a. Cette face opposée 2c de la cartouche 2 présente une saillie creuse centrale 2d dont le conduit interne est fermé par un élément de rupture formant obturateur 2e. Une saignée circulaire d'affaiblissement 2f est formée entre la saillie creuse 2d et l'obturateur 2e, pour permettre de séparer l'obturateur 2e de la saillie creuse 2d. Un conduit de distribution 3 est fixé de manière étanche par soudage sur la saillie creuse 2d.

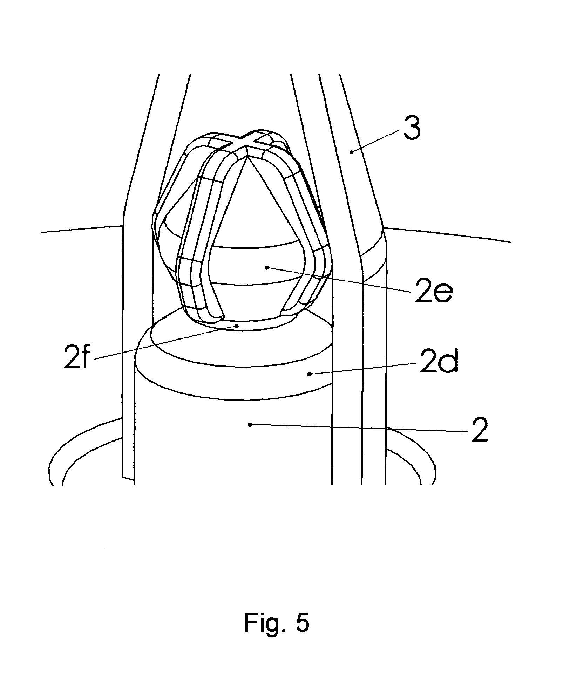

Comme illustré par la figure 5, la face externe de l'obturateur 2e n'est pas circulaire, pour permettre le passage du liquide de la cartouche 2 lorsque l'obturateur a été séparé de la saillie creuse 2d par rupture manuelle ou mécanique, empêchant à la surface externe de l'obturateur 2e d'être en contact sur toute sa surface avec la paroi du conduit de distribution 3 du liquide d'injection 1, ce qui aurait pour effet d'empêcher l'écoulement. La rupture mécanique de l'obturateur 2e peut être obtenue par une partie excentrique (non représentée) ménagée sur le couvercle 9b et qui rompt l'obturateur lors de la rotation de la fixation à baïonnette du couvercle 9b à l'extrémité du corps tubulaire 9a.

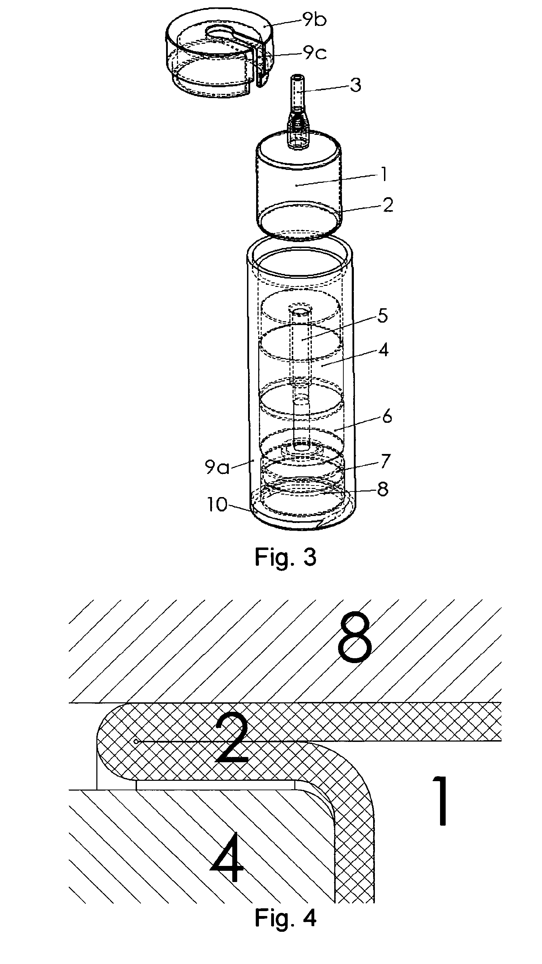

De préférence, la partie avant 4a du piston 4 présente un rayon légèrement plus petit que la partie arrière 4b. Cette différence de rayon est au moins égale au double de l'épaisseur de la paroi latérale 2b de la cartouche 2 pour permettre à celle-ci de se replier sur elle-même, comme illustré par les figures 2 et 4. Avantageusement, comme illustré par la figure 4, pour éviter le coincement du piston 4, le rayon du segment avant 4a du piston 4 est légèrement plus petit que le double de l'épaisseur de la paroi latérale 2b de la cartouche 2.

De préférence, des moyens de coulissement (rainure et saillie rectilignes parallèles à l'axe de révolution du corps tubulaire 9a) seront ménagés sur la face interne du corps tubulaire 9a, respectivement sur la face latérale du piston 4 pour empêcher la rotation du piston 4.

De son côté l'épaisseur de la paroi 2b est choisie en fonction de la matière plastique utilisée. Typiquement, l'épaisseur de la paroi 2b de la cartouche 2 est de l'ordre de 0,25 mm pour du PP ou du PE. Elle peut cependant varier entre 0,05 mm et 1 mm en fonction du matériau employé et de l'application.

Pour faciliter la mise en place de la cartouche 2 dans le corps tubulaire 9a, la face interne de ce corps tubulaire peut comporter des rainures parallèles à l'axe longitudinal du corps 9a, réduisant ainsi la surface de frottement et permettant d'augmenter les tolérances de fabrication en diamètre.

La cartouche peut avantageusement être extrudée et comporter des surfaces d'extrémités 2a, 2c bombées permettant d'éviter de trop gros efforts d'introduction de la cartouche 2 dans le corps tubulaire 9a et d'augmenter les tolérances de fabrications. Une précontrainte de 0.05% à 1% en diamètre permet d'éviter le risque de collapsus du réservoir et une injection non contrôlée du liquide d'injection au cas où la ligne de perfusion serait mise sous dépression (par exemple 1 m de colonne d'eau).

Un capteur de force (non représenté) peut être disposé entre la face avant du piston 4 et la face 2a de la cartouche pour mesurer la pression dans le conduit d'injection 3 et par exemple donner une alarme d'occlusion.

La position du piston 4 et donc le volume injecté peut être détectée, dans le cas d'un moteur 6 à courant continu, sans ballets, par trois capteurs à effet Hall et dans le cas d'un moteur pas à pas, par un codeur optique.

L'ensemble des informations est transmis à l'électronique de commande 7.

Le réservoir 2 peut être avantageusement fabriqué par la technologie bien connue sous le terme anglais de « Blow-Fill-Seal » soit soufflée-remplie-scellée, en une seule pièce, co-extrudée avec une barrière vapeur pour éviter la perte de liquide durant les 3 ans durant lesquels la cartouche 2 doit pouvoir être stockée. On peut aussi plus classiquement fabriquer la cartouche en deux pièces avec une des parois 2a ou 2c rapportée, soudé ou collé.

Le moteur 6 d'entraînement du piston 4 par l'intermédiaire de l'arbre fileté 5 suit un programme de débit et/ou volume préétablis, programmable par l'utilisateur, le patient ou le médecin, selon les applications.

Le système est de préférence autonome étant avantageusement alimenté par la pile 8 ou par un accumulateur rechargeable et contrôlé par le circuit du module électronique 7. En raison de sa petite taille, le dispositif de distribution est ambulatoire. Il peut facilement être fixée dans les habits du patient.

L'invention trouve une autre application dans les salles d'opération pour générer, à l'aide de plusieurs dispositifs de distribution, le mélange anesthésique. Cette application est connue sous l'appellation « pousse-seringues ». Un empilement de 5 à 10 dispositifs de distribution est possible, sans prendre beaucoup de place dans la zone de travail de l'anesthésiste. Ces dispositifs modulaires peuvent être contrôlées par un ordinateur et un écran central.

Les cartouches peuvent être identifiées par un système de code barre qui permet de vérifier que le produit contenu dans la cartouche correspond à l'injection à effectuer. De préférence, le code barre est imprimé en relief sur la cartouche 2. Le dispositif de distribution lit le code barre et ne peut fonctionner que si le produit correspondant est celui pour lequel le dispositif de distribution est programmé. On ne peut donc pas injecter un autre produit que celui auquel le dispositif est programmé.

Pour permettre l'injection de volume de 10 à 50 ml sans changer de boîtier, il est avantageux de prévoir un seul diamètre pour les cartouches 2 d'une contenance de 10-20 et 50 ml par exemple, avec les longueurs de cartouches variables en conséquence. Si on choisit un diamètre d'environs 37 mm, l'utilisateur a une vision du volume V perfusé par le déplacement longitudinal L du piston 4.

Par exemple, pour un diamètre commun de 37 mm

| | | | | |
|---|---|---|---|---|
| V = | 10 | 20 | 50 | ml |
| L = | 10 | 20 | 50 | mm |

Bien évidemment, la cartouche de liquide 2 pourrait aussi être utilisée avec un dispositif dans lequel le piston 4 est entraîné manuellement, telle qu'une seringue d'anesthésie utilisée en dentisterie. Typiquement, une telle seringue peut avoir un diamètre de 9 mm environ et 50 mm de longueur environ.

## Revendications

1. Dispositif de distribution d'un liquide d'injection, comprenant un corps tubulaire (9a) présentant à une extrémité une ouverture de sortie (2d) et dans lequel une cartouche (2) hermétique et jetable contient le liquide d'injection (1), **caractérisé en ce que** ce corps tubulaire (9a) comprend un piston (4) pour expulser le liquide d'injection (1) de la cartouche (2) à travers l'ouverture de sortie (2d), au moins une partie (2b) de la paroi de la cartouche (2) étant déformable sous la pression exercée par le piston (4), cette cartouche (2) comprenant un élément de raccordement étanche (2d) pour un conduit de distribution (3) et un élément de rupture (2e) pour mettre en communication l'intérieur de la cartouche (2) avec le conduit de distribution (3), le corps tubulaire (9a) comportant une partie amovible (9b) pour la mise en place et l'enlèvement de la cartouche (2).

2. Dispositif de distribution selon la revendication 1, dans lequel la cartouche (2) comporte une paroi latérale (2b), adjacente à la paroi tubulaire (9a) dudit corps, fermée à chaque extrémité par une paroi d'extrémité (2a, 2c) et constituant la paroi déformable susceptible d'être repliée sur elle-même par le déplacement du piston (4), le segment (4a) de la paroi latérale de ce piston (4), adjacent à la cartouche (2), présentant un rayon réduit de la valeur d'au moins deux fois l'épaisseur de la paroi latérale (2b) de la cartouche (2).

3. Dispositif de distribution selon l'une des revendications précédentes, dans lequel ledit corps (9a) renferme un moteur électrique (6) pour l'entraînement du piston (4).

4. Dispositif de distribution selon la revendication 3, dans lequel ledit corps (9a) renferme une batterie électrique (8) pour alimenter le moteur électrique (6).

5. Dispositif de distribution selon l'une des revendications précédentes, dans lequel l'élément de raccordement (2d) comporte une saillie creuse située au centre d'une des parois d'extrémité (2c) de la cartouche (2) et qui se termine par un obturateur (2e), une saignée circulaire d'affaiblissement (2f) étant formée entre la saillie creuse (2d) et l'obturateur (2e).

6. Dispositif de distribution selon la revendication 5, dans lequel la face externe de l'obturateur (2e) est de forme non circulaire pour permettre l'écoulement du liquide dans le conduit de distribution (3) après la séparation de l'obturateur (2e) de l'élément de raccordement (2d).

7. Dispositif selon la revendication 1, dans lequel la partie amovible du corps tubulaire (9a) est formée par un couvercle (9b) relié à l'extrémité ouverte du corps tubulaire (9a) par une fixation à baïonnette, ce couvercle (9b) comportant une ouverture radiale (9c) pour permettre le passage de l'élément de raccordement (2d) relié de manière étanche au conduit de distribution (3).

8. Dispositif selon la revendication 1 dans laquelle le piston (4) est un piston à entraînement manuel d'une seringue d'anesthésie utilisée en dentisterie.

9. Procédé de fabrication de la cartouche hermétique et jetable contenant le liquide d'injection selon la revendication 1, selon lequel la cartouche (2) est réalisée par la technique d'extrusion soufflage, remplissage et scellage.
